(19) Europäisches Patentamt
European Patent Office
Office européen des brevets

(11) Veröffentlichungsnummer: **0 276 674 A1**

# (12) EUROPÄISCHE PATENTANMELDUNG

(21) Anmeldenummer: **88100279.4**

(22) Anmeldetag: **12.01.88**

(51) Int. Cl.4: **A61K 9/00**, A61K 47/00, A61K 31/44

(30) Priorität: **24.01.87 DE 3702105**

(43) Veröffentlichungstag der Anmeldung: **03.08.88 Patentblatt 88/31**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Hoederath, Wolfgang, Dr.**
**Josefsstrasse 7**
**D-5253 Lindlar-Linde(DE)**
Erfinder: **Ahr, Hans Jürgen, Dr.**
**Am Rohm 82**
**D-5600 Wuppertal 1(DE)**
Erfinder: **Bühner, Klaus, Dr.**
**Hainstrasse 92**
**D-5600 Wuppertal 1(DE)**
Erfinder: **Hegasy, Ahmed, Dr.**
**Tempelhofer Strasse 57**
**D-5090 Leverkusen 1(DE)**
Erfinder: **Winter, Manfred, Dr.**
**Roggendorfstrasse 49**
**D-5000 Köln 80(DE)**

## (54) Parenterale Lösung.

(57) Parenterale Lösung, enthaltend

a) einen schwer löslichen Arzneimittelwirkstoff
b) ein Lösungsmittel bestehend aus
(i) 5 - 100 G/V % eines organischen Lösungsmittels und
(ii) 0 - 95 G/V % Wasser
c) eine 0.5 - 30 G/V %ige wässrige Lösung eines Humanserumproteins sowie übliche Hilfs-und/oder Trägerstoffe,

wobei pro ein Gewichtsteil Arzneimittelwirkstoff 1 bis 40.000 Gewichtsteile des Lösungsmittels b) und 1 bis 1.000.000 Gewichtsteile der Humanserumproteinlösung c) vorliegen;
sowie ein Verfahren zu ihrer Herstellung.

EP 0 276 674 A1

## Parenterale Lösung

Die parenterale Applikation von Arzneistoffen, im wesentlichen die intravenöse Applikation, ist nur in gelöster Form möglich. Daher bereitet die Formulierung von Injektions-und Infusionslösungen bei Arzneistoffen geringer Wasserlöslichkeit regelmäßig Schwierigkeiten.

Im Falle unzureichender Löslichkeit in Wasser wurden bislang bei der Formulierung parenteraler Lösungen schwer löslicher Arzneistoffe organische Lösungsmittel wie Propylenglykol, Polyethylenglykol, Ethanol, Glycerin-Polyethylenglykolricinoleat (Cremophor® EL) oder Polyoxyethylensorbitanfettsäureester (Tween®) zur Erhöhung der Löslichkeit zugesetzt. Die Effektivität dieser Maßnahme wird allerdings dadurch begrenzt, daß Lösungsmittel nur in niederen Konzentrationen angewendet werden können, da höhere Konzentrationen zu unerwünschten Nebenwirkungen wie Injektionsschmerz, Thrombophlebitis und Venenverödung führen. Darüber hinaus führen einige Lösungsmittel zu Nebenwirkungen wie anaphylaktische Schocks und Hämolyse.

Eine andere Möglichkeit der Lösungsvermittlung von Arzneistoffen geringer Wasserlöslichkeit besteht darin, den Arzneistoff in der Fettphase in einer Emulsion zu lösen (US 4 073 943). Dieses Verfahren setzt allerdings eine sehr gute Löslichkeit des Arzneistoffs in physiologisch verträglichen Ölen wie Sojabohnenöl voraus, die nur in den seltensten Fällen gewährleistet ist.

Die in US 4 158 707 beschriebene Lösungsvermittlung mit einer Kombination aus Gallensäure und Lipoid hat den Nachteil einer begrenzten Haltbarkeit der Lösungsvermittler, insbesondere bei höheren Temperaturen, sowie von Nebenwirkungen wie Erbrechen, Hämolyse und Cholestasis bei Gaben in höheren Dosen.

Die vorliegende Erfindung betrifft nunmehr parenterale Lösungen von schwer löslichen Arzneimitteln, die Humanserumproteine enthalten, wobei die Humanserumproteine als Kristallisationsinhibitoren dienen.

Parenterale Lösungen sind hierbei im wesentlichen intravenöse Applikationen mit Arzneistoffen, im besonderen Injektions-und Infusionlösungen. Für die erfindungsgemäßen parenteralen Lösungen verwendet man im allgemeinen wirkstoffhaltige Konzentrate in geeigneten organischen Lösungsmitteln wie 1,2-Propylenglykol, Glycerin, Ethanol, Polyethylenglykolen mit mittleren Molgewichten zwischen 200 und 600 und Tetrahydrofurfurylalkoholpolyethylenglykolether im Gemisch mit Wasser, die mit wäßrigen Humanserumprotein-Lösungen vor Applikation verdünnt werden. Als Humanserumprotein-Lösungen werden vorzugsweise Humanalbumin-Lösungen (USP XXI) oder Plasma-Protein-Fraktion-Lösungen (USP XXI) eingesetzt, wobei letztere in aller Regel die Wirkstoffpräzipitation länger dauernd und auch bei höherer Konzentration des Arzneistoffes verzögern. Die Serumproteine können neben Albumin auch $\alpha$-, $\beta$-und $\gamma$-Globuline enthalten.

Die Erfindung betrifft somit eine parenterale Lösung enthaltend

a) einen schwer löslichen Arzneimittelwirkstoff

b) ein Lösungsmittel bestehend aus

(i) 5 - 100 G/V % eines organischen Lösungsmittels oder eines Gemisches organischer Lösungsmittel und

(ii) 0 - 95 G/V % Wasser

c) eine 0,5 - 30 G/V %ige wässrige Lösung eines Humanserumproteins sowie übliche Hilfs-und oder Trägerstoffe,

wobei pro ein Gewichtsteil Arzneimittelwirkstoff 1 bis 40.000 Gewichtsteile, bevorzugt 25 bis 30.000 Gewichtsteile des Lösungsmittels b) und 1 bis 1.000.000 Gewichtsteile, bevorzugt 50 bis 40.000 Gewichtsteile der Humanserumproteinlösung c) vorliegen.

Die erfindungsgemäß verwendbaren schwerlöslichen Arzneimittelwirkstoffe besitzen im allgemeinen eine Löslich keit in Wasser zwischen 1 µg und 10 g, bevorzugt zwischen 10 µg und 1 g pro Liter Wasser. Beispielhaft seien Dihydropyridinverbindungen und Pyrazolone und Muzolimin aufgeführt.

Von ganz besonderer Bedeutung sind die Dihydropyridinverbindungen, insbesondere die mit folgender allgemeiner Formel

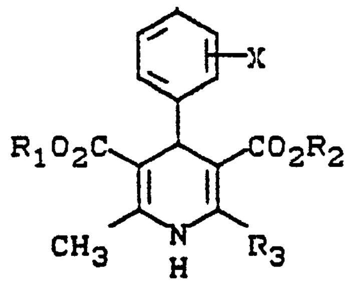

(I)

in der

$R_1$ $C_1$-$C_4$-Alkyl, gegebenenfalls substituiert durch $C_1$-$C_3$ Alkoxy,

$R_2$ $C_1$-$C_{10}$-Alkyl, gegebenenfalls substituiert durch $C_1$-$C_3$-Alkoxy, Trifluormethyl, N-Methyl-N-benzylamino,

$R_3$ $C_1$-$C_4$-Alkyl, Cyano, Hydroxymethyl•und

X 2-bzw. 3-Nitro, 2,3-Dichlor, 2,3 Ringglied bestehend aus =N-O-N=,

bedeuten.

Ganz besonders in Betracht kommen die Verbindungen der folgenden Tabelle:

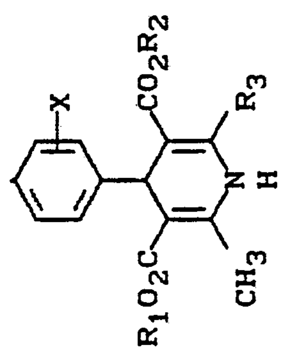

(I)

| Nr. | X | $R^1$ | $R^2$ | $R^3$ | Generic |
|---|---|---|---|---|---|
| 1 | $2\text{-}NO_2$ | $CH_3$ | $CH_3$ | $CH_3$ | Nifedipin |
| 2 | $3\text{-}NO_2$ | $nPrOCH_2CH_2$ | $nPrOCH_2CH_2$ | $CH_3$ | Niludipin |
| 3 | $3\text{-}NO_2$ | $C_2H_5$ | $CH_3$ | $CH_3$ | Nitrendipin |
| 4 | $2\text{-}NO_2$ | $CH_3$ | $(CH_3)_2CHCH_2$ | $CH_3$ | Nisoldipin |
| 5 | $3\text{-}NO_2$ | $CH(CH_3)_2$ | $(CH_2)_2\text{-}O\text{-}CH_3$ | $CH_3$ | Nimodipin |
| 6 | $3\text{-}NO_2$ | $C_2H_5$ | $C_{10}H_{21}(n)$ | $CH_3$ | |
| 7 | 2-Cl | $CH_3$ | $CH_2\text{-}CF_3$ | $CH_3$ | |
| 8 | 2-Cl | $C_2H_5$ | $CH_2\text{-}CF_3$ | $CH_3$ | |
| 9 | $3\text{-}NO_2$ | $CH(CH_3)_2$ | $n\text{-}PrO\text{-}CH_2\text{-}CH_2$ | $CH_3$ | |
| 10 | $3\text{-}NO_2$ | $CH_3$ | $C_6H_5CH_2N(CH_3)CH_2CH_2$ | $CH_3$ | Nicardipin |
| 11 | $2,3\text{-}Cl_2$ | $C_2H_5$ | $CH_3$ | $CH_3$ | Felodipin |
| 12 | 2,3=N-O-N= | $C_2H_5$ | $C_2H_5$ | $CH_3$ | |
| 13 | 2,3=N-O-N= | $CH_3$ | $CH(CH_3)_2$ | $CH_3$ | |
| 14 | $3\text{-}NO_2$ | $C_2H_5$ | $C_2H_5$ | $CH_2OH$ | |
| 15 | $3\text{-}NO_2$ | $CH_3$ | $CH_3$ | CN | |
| 16 | $3\text{-}NO_2$ | $CH_3$ | $(CH_2)_3\text{-}(CF_2)_5\text{-}CF_3$ | $CH_3$ | |

n-Pr = n-Propyl

Darüberhinaus sei als Dihydropyridinverbindung noch 2-Methyl-4-(4-oxo-2-phenyl-4H-thiochromen-8-yl)-5-oxo-1,4,5,7-tetrahydrofuro-[3,4-b]-pyridin-3-carbonsäureethyl oder -methylester erwähnt.

Die erfindungsgemäß einsetzbaren Humanalbumin-oder Plasma-Protein-Fraktion-Lösungen sind im Handel in Form 5, 20 oder 25 %iger Lösungen erhältlich und sind in vielen Pharmakopöen beschrieben, beispielsweise in USP XXI, BP 80 sowie in Remington's Pharmaceutical Sciences. Mack Publishing Company. Easton Pennsylvania.

Die parenteral Lösungen können unter anderem Hilfs-und oder Trägerstoffe enthalten, wie N-Acetyl-dl-tryptophan, Caprylat, Acetat, Citrat, Glukose sowie Elektrolyte wie Natrium, Kalium, Calcium, Magnesium und Chlorid, Phosphate, Hydrogencarbonat

Außerdem:

Säuren, Basen oder Puffersubstanzen zur pH-Einstellung, Salze, Zucker oder mehrwertige Alkohole zur Isotonisierung, Konservierungsmittel wie Benzylalkohol, Chlorbutanol, Antioxidantien wie Sulfite, Acetylcystein oder Ascorbinsäure.

Die erfindungsgemäße parenterale Lösung kann hergestellt werden, indem man zunächst den Arzneimittelwirkstoff in einem organischen Lösungsmittel auflöst und gegebenenfalls zusätzlich Wasser ergänzt. Dieses Konzentrat wird anschließend filtriert, abgefüllt und unmittelbar vor der Applikation mit Humanserumproteinlösung verdünnt.

Die so erhaltenen Lösungen sind zwar häufig deutlich übersättigt, die Wirkstoffpräzipitation wird aber verzögert und tritt meist erst nach einigen Stunden auf.

## Versuchsbeispiele

Injektionslösungen entsprechend dieser Erfindung können wie folgt hergestellt werden:

1. - Konzentrat des unlöslichen Arzneistoffes

2-Methyl-4-(4-oxo-2-phenyl-4 H-thiochromen-8-yl)-5-oxo-1,4,5,7-tetrahydrofuro [3,4-b] pyridin-3-carbonsäureethylester wird zu 0,5 in einer Mischung aus 600 g Polyethylenglykol 400 und 200 g Ethanol gelöst unter Rühren und Erwärmen. Nach dem Abkühlen auf 20°C wird der Verdunstungsverlust an Ethanol ergänzt und mit Wasser für Injektionszwecke auf 1 Liter aufgefüllt.

-Fertige Injektionslösung

2 ml des oben beschrieben Konzentrates werden mit 8 ml Humanalbumin 5 % oder Plasma-Protein-Fraktion 5 % versetzt und vermischt.

Die so erhaltene applikationsfertige, übersättigte Lösung ist über einen Zeitraum von mehreren Stunden klar und praktisch frei von Partikeln, da eine Auskristallisation durch die Verwendung der Proteinlösung als Verdünnungsmedium verzögert wird. Wird das Wirkstoffkonzentrat mit der entsprechenden Menge Wasser verdünnt, so tritt eine sofortige Ausfällung des Arzneistoffes ein.

2. - Konzentrat des unlöslichen Arzneistoffes

1 g Nifedipin wird unter Erwärmen in einer Mischung auf 250 g Ethanol und 250 g Polyethylenglykol 400 gelöst. Nach Abkühlen und Ergänzen der verdunsteten Alkoholmenge wird mit Wasser für Injektionszwecke auf 600 ml aufgefüllt.

-Fertige Injektionslösung,

0,6 ml dieses Konzentrates werden mit 2,4 ml Humanalbumin 5 %ig oder 2,4 ml Plasma-Protein-Fraktion 5 %ig versetzt und vermischt.

Es resultiert eine über mehrere Stunden Klare Nifedipin-Injektionslösung.

3. - Konzentrat des unlöslichen Arzneistoffes

10 g Nisoldipin werden in einer Mischung aus 3.000 g Ethanol und 3.000 g Polyethylenglykol 400 unter Rühren und Erwärmen gelöst. Nach dem Abkühlen auf Raumtemperature wird der Verdunstungsverlust an Ethanol ergänzt und mit Wasser für Injektionszwecke auf 7 l aufgefüllt.

-Fertige Injektionslösung

0,35 ml des oben beschriebenen Konzentrates werden mit 2,7 ml Humanalbumin 5 % oder Plasma-Protein-Fraktion 5 % versetzt und vermischt.

Es ergibt sich eine über mehrere Stunden klare Nisoldipin Injektionslösung.

4. -Konzentrat des unlöslichen Arzneistoffes

10 g Nitrendipin werden in einer Mischung aus 4.000 g Ethanol und 4.000 g Polyethylenglykol 400 unter Rühren und Erwärmen gelöst. Nach dem Abkühlen auf Raumtemperatur wird der Verdunstungsverlust an Ethanol ergänzt und mit Wasser für Injektionszwecke auf 10 l aufgefüllt.

-Fertige Injektionslösung

1,0 ml des oben beschriebenen Konzentrates werden mit 4.0 ml Humanalbumin 5 % oder Plasma-Protein-Fraktion 5 % versetzt und vermischt.

Es ergibt sich eine über mehrere Stunden stabile Nitrendipin Injektionslösung.

5. -Konzentat des unlöslichen Arzneistoffes

60 g Muzolimin werden in einer Mischung auf 2.000 g Ethanol und 2.000 g Polyethylenglykol 400 unter Rühren gelöst. Anschließend füllt man mit Wasser für Injektionszwecke auf 5 l auf.

-Fertige Injektionslösung

2,5 ml des oben beschriebenen Konzentrats werden mit 2,5 ml Humanalbumin 5 % oder Plasma-Protein-Fraktion 5 % versetzt und vermischt.

Es entsteht eine über mehrere Stunden klare Muzolimin Injektionslösung.

## Ansprüche

1. Parenterale Lösung, enthaltend
a) einen schwer löslichen Arzneimittelwirkstoff
b) ein Lösungsmittel bestehend aus
(i) 5 - 100 G/V % eines organischen Lösungsmittels oder eines Gemisches organischer Lösungsmittel und
(ii) 0 - 95 G/V % Wasser
c) eine 0,5 - 30 G/V %ige wässrige Lösung eines Humanserumproteins
sowie übliche Hilfs-und/oder Trägerstoffe,
wobei pro ein Gewichtsteil Arzneimittelwirkstoff 1 bis 40.000 Gewichtsteile des Lösungsmittels b) und 1 bis 1.000.000 Gewichtsteile der Humanserumproteinlösung c) vorliegen.

2. Parenterale Lösung gemäß Anspruch 1, enthaltend 25 bis 30.000 Gewichtsteile des Lösungsmittels b) und 50 bis 40.000 Gewichtsteile der Humanserumproteinlösung c) pro 1 Gewichtsteil Arzneimittelwirkstoff.

3. Parenterale Lösung gemäß Anspruch 1 und 2, enthaltend einen Arzneimittelwirkstoff mit einer Löslichkeit zwischen 1 µg und 1 g pro Liter Wasser.

4. Parenterale Lösung gemäß Ansprüchen 1 - 3, enthaltend als Arzneimittelwirkstoff eine Dihydropyridinverbindung oder Pyrazolonverbindung.

5. Verfahren zur Herstellung einer parenteralen Lösung, enthaltend
a) einen schwer löslichen Arzneimittelwirkstoff
b) ein Lösungsmittel bestehend aus
(i) 5 - 100 G/V % eines organischen Lösungsmittels oder eines Gemisches organischer Lösungsmittel und
(ii) 0 - 95 G/V % Wasser
c) eine 0,5 - 30 G/V %ige wässrige Lösung eines Humanserumproteins
sowie übliche Hilfs-und/oder Trägerstoffe,
wobei pro ein Gewichtsteil Arzneimittelwirkstoff 1 bis 40.000 Gewichtsteile des Lösungsmittels b) und 1 bis 1.000.000 Gewichtsteile der Humanserumproteinlösung c) vorliegen,
dadurch gekennzeichnet, daß man den Arzneimittelwirkstoff in einem Lösungsmittel löst und anschließend eine Lösung eines Humanserumproteins zusetzt.

Europäisches Patentamt

# EUROPÄISCHER RECHERCHENBERICHT

Nummer der Anmeldung

EP 88 10 0279

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 123 850 (THE BOARD OF GOVERNORS OF WAYNE STATE UNIVERSITY)<br>* Seite 9, Zeilen 19-27; Seite 14, Zeile 10; Ansprüche *<br>--- | 1-5 | A 61 K 9/00<br>A 61 K 47/00<br>A 61 K 31/44 |
| Y | EP-A-0 126 315 (BAYER AG)<br>* Ansprüche *<br>--- | 1-5 | |
| Y | DE-C- 427 669 (F.E. MERCK)<br>* Insgesamt *<br>--- | 1 | |
| X,Y | EP-A-0 140 255 (SUMITOMO CHEMICAL CO.)<br>* Ansprüche 1,3,4,8,12 *<br>----- | 1-5 | |
| | | | **RECHERCHIERTE SACHGEBIETE (Int. Cl.4)** |
| | | | A 61 K |

Der vorliegende Recherchenbericht wurde für alle Patentansprüche erstellt

| Recherchenort | Abschlußdatum der Recherche | Prüfer |
|---|---|---|
| DEN HAAG | 17-04-1988 | BERTE M.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)